# EUROPÄISCHE PATENTSCHRIFT

(11) **EP 0 597 068 B1**
(45) Veröffentlichungstag und Bekanntmachung des Hinweises auf die Patenterteilung: **04.02.1998**
(21) Anmeldenummer: 93911748.7
(22) Anmeldetag: 11.05.1993
(51) Int. Cl.: G01N 33/543, G01N 33/80, C12Q 1/68, G01N 33/577, B03C 1/00

(54) **VERFAHREN ZUR PRÄNATALDIAGNOSTIK GENETISCHER ANOMALIEN**
PROCESS FOR THE PRENATAL DIAGNOSIS OF GENETIC ABNORMALITIES
PROCEDE DE DIAGNOSTIC PRENATAL D'ANOMALIES GENETIQUES

(30) Priorität: 16.05.1992 DE 4216345; 09.07.1992 DE 4222573
(43) Veröffentlichungstag der Anmeldung: 18.05.1994
(73) Patentinhaber: AHLERT, Dorothee, 4149 Münster (DE); HOLZGREVE, Wolfgang, D-48149 Münster (DE); GARRITSEN, Hendrikus, Stephanus, Paulus, D-48149 Münster (DE)
(72) Erfinder: AHLERT, Dorothee, 4149 Münster (DE); HOLZGREVE, Wolfgang, D-48149 Münster (DE); GARRITSEN, Hendrikus, Stephanus, Paulus, D-48149 Münster (DE)
(74) Vertreter: Habbel, Hans-Georg, Dipl.-Ing.
(86) Internationale Anmeldenummer: DE9300427
(87) Internationale Veröffentlichungsnummer: WO9323754

(56) Entgegenhaltungen:
- EP-A- 0 079 696
- WO-A-90/06509
- JOURNAL OF IMMUNOLOGICAL METHODS. Bd. 131, 1990, NEW YORK US Seiten 147 - 149 N. M. BHAT ET AL. 'One step separation of human fetal lymphocytes from nucleated red blood cells' in der Anmeldung erw hnt
- CHEMICAL ABSTRACTS, vol. 113, no. 17, 22. Oktober 1990, Columbus, Ohio, US; abstract no. 150231, G. N. P. VAN MUIJEN ET AL. 'Monoclonal antibody PAL-M1 recognizesthe transferrin receptor and is a progression marker in melanocytic lesions' Seite 579 ;Spalte 1 ;
- CHEMICAL ABSTRACTS, vol. 114, no. 21, 27. Mai 1991, Columbus, Ohio, US; abstract no. 205143, P. BJOERCK ET AL. 'Expression of CD40 and CD43 during activation of human B lymphocytes' Seite 632 ;Spalte 1 ;
- BIOLOGICAL ABSTRACTS vol. 94, no. 4 , 15. August 1992, Philadelphia, PA, US; abstract no. 37307, W. HOLZGREVE ET AL. 'Fetal cells in the maternal circulation.' Seite AB-241 ;
- BIOLOGICAL ABSTRACTS vol. 94, no. 4 , 15. August 1992, Philadelphia, PA, US; abstract no. 37313, D. GAENSHIRT-AHLERT 'Magnetic cell sorting and the transferrin receptor as potential means of prenatal diagnosis from maternal blood' Seite AB-242 ;

## Beschreibung

Die Erfindung betrifft ein Verfahren zur Pränataldiagnostik genetischer Anomalien.

Aus der FR 2 657 167 ist ein nichtdiagnostisches Screening-Verfahren bekannt, bei dem für die Screeninguntersuchungen nur mütterliches Serum verwendet wird. Auf diese Weise kann ein altersbedingtes Risiko für Morbus Down rechnerisch modifiziert werden, wobei durch diese Untersuchungen jedoch nur ein relatives Risiko für das Down Syndrom geschätzt werden kann.

Aus der WO-A-90/06509 ist ein Verfahren zur Pränataldiagnostik mit aus der mütterlichen Zirkulation isolierten fetalen Zellen bekannt. Die Anwendung eines Mehrfach-Dichte-Gradienten geht aus dieser Druckschrift nicht hervor.

Im übrigen ist es seit ca. 20 Jahren möglich, vorgeburtlich genetische Anomalien zu diagnostizieren. Die zur Zeit zur Verfügung stehenden Techniken zur Aspiration kindlichen Gewebes während der Schwangerschaft sind die Fruchtwasserpunktion (Amniozentese) und die sog. Chorionzottenbiopsie. Die Chorionzottenbiopsie kann bereits ab der 8., die Amniozentese ab der 14. Schwangerschaftswoche durchgeführt werden. Die Invasivität dieser konventionellen Methoden bedingt jedoch, daß Eingriffsrisiken für den Feten (z. B. Fehlgeburt oder Verletzung) aber auch für die Mutter (z. B. Entzündungen in der Gebärmutter) bestehen. Eine randomisierte Kontrollstudie wies nach, daß sogar durch eine Amniozentese, die von vielen als die sicherere Aspirationstechnik angesehen wird, das Risiko eines Spontanabortes eingriffsbedingt um ca. 1 % steigt (Holzgreve W.,Miny P : Genetic aspects of fetal disease. Semin Perinatal 13: 260, 1989). Deshalb wird zur Zeit eine Untersuchung auf eine spontan auftretende sogenannte kindliche Trisomie (überzähliges Chromosom) nur bei Müttern mit erhöhtem Risiko, z. B. erhöhtem mütterlichen Alter durchgeführt. Ein invasiver Eingriff wird in Deutschland erst bei Schwangeren ab dem 36. Lebensjahr durchgeführt, weil das Eingriffsrisiko bei jüngeren Schwangeren im Normalfall höher ist, als die Wahrscheinlichkeit des Auftretens einer kindlichen Trisomie. Deshalb besteht seit vielen Jahren eine starke Motivation zur Entwicklung einer weniger invasiven Technik zur Pränataldiagnostik. Obwohl eine Vielzahl von Versuchen in der Vergangenheit unternommen wurde, diese Hoffnung zu verwirklichen, war keine der bisher versuchten Techniken erfolgreich.

Vermutlich gelangen im Laufe einer Schwangerschaft einige wenige kindliche Zellen, als Folge fetomaternaler Transfusion, in den mütterlichen Kreislauf. Das Verhältnis kindlicher zu mütterlichen kernhaltigen Zellen im Blut einer Schwangeren ist aber extrem niedrig und wird zur Zeit auf ca. 1 in 10⁹ bis 1 in 10¹¹ Zellen geschätzt (Holzgreve W, Gänshirt-Ahlert D, Burschky M et al.: Detection of fetal DNA in maternal blood by PCR. Lancet 335: 1220, 1990). Wegen dieser hohen Verdünnung kindlicher Zellen ist es ohne vorherige Anreicherung bisher noch nicht gelungen, das genetische Material des Feten aus mütterlichem Blut reproduzierbar nachzuweisen (Holzgreve W, Garritsen HSK,Gänshirt-Ahlert D: Fetal cells in maternal circulation. J Reprod Med 37 (5): 410, 1992, nicht vorveröffentlicht)

Wenn es möglich wäre, diese fetalen Zellen aus der mütterlichen Zirkulation zu isolieren, wäre durch eine einfache Blutentnahme der Zugang zu einer Pränataldiagnostik möglich. Hierdurch würde das durch den konventionellen Eingriff bedingte Risiko für Mutter und Kind entfallen. Zusätzlich würde eine Blutentnahme gegenüber der heute üblichen Amniozentese bzw. Chorionzottenbiopsie eine erhebliche Kostensenkung bedeuten. Amniozentesen werden nur von besonders dafür ausgebildeten Frauenärzten durchgeführt und die neuere Chorionzottenbiopsie wird zur Zeit in Deutschland nur in wenigen speziell dafür eingerichteten Zentren angeboten. Da alle kernhaltigen Zellen die gesamte genetische Information enthalten, können aus kindlichen Blutzellen nach erfolgreicher Isolation aus mütterlichem Blut dieselben genetischen Aberrationen nachgewiesen werden, wie nach konventioneller Aspiration fetaler Zellen.

Der Erfindung liegt die Aufgabe zugrunde, genetische Anomalien pränatal nachzuweisen.

Die Lösung der Aufgabe besteht aus einer Kombination folgender Schritte:
a) Voranreicherung kindlicher Zellen in Form von nukleierten Erythrocyten aus mütterlichem Vollblut durch einen Dreifach-Ficoll-Gradienten,
b) Markierung der kindlichen Zellen mit einem monoklonalen Antikörper und/oder Markierung mütterlicher Zellen mit entsprechenden spezifischen Antikörpern mit magnetischen Beads und Anreicherung der markierten fetalen Zellen bzw. Depletion der mütterlichen Zellen durch magnetische Separation,
c) Nachweis kindlicher genetisch bedingter Anomalien durch molekulargenetische Nachweismethoden.

Die Konzentration der fetalen Zellen kann also entweder durch deren Anreicherung erfolgen oder über die Depletion der müttlichen Zellen.

Vorteilhaft können die kindlichen Zellen mit einem monoklonalen Antikörper gegen den Transferrinrezeptor markiert werden.

Vorzugsweise wird als monoklonaler Antikörper ein spezifischer "anti - CD71" (Becton-Dickenson) eingesetzt.

Der Nachweis kindlicher genetisch bedingter Anomalien erfolgt vorzugsweise als Nachweis kindlicher Chromosomenstörungen durch Fluoreszenz in situ Hybridisierung mit chromosomen-spezifischen DNA Sonden.

Die Anreicherung der markierten fetalen Zellen durch einen magnetisch aktivierten Zellsorter (MACS) hat gute Ergebnisse erbracht und ist preisgüntig. Sie kann jedoch alternativ beispielsweise auch mit Hilfe sogenannter "Dynabeads" erfolgen, bei denen mit einem offenen Reagenzgefäß und einem außerhalb des Reagenzgefäßes angebrachten Magneten gearbeitet wird.

Die Untersuchung der Zellzusammensetzung fetalen Blutes zeigt, daß nukleierte Erythrocyten in der frühen Entwicklung (Bis zur 20. Schwangerschaftswoche) den überwiegenden Anteil der kernhaltigen Zellen in der fetalen Zirkulation ausmachen. Erst im späteren Verlauf der Schwangerschaft nimmt die Anzahl der weißen Blutkörperchen (Lymphocyten) zu. Dies beruht auf der speziellen Ontogenese der fetalen Blutbildung, die im Laufe der Schwangerschaft in verschiedenen Entwicklungsphasen in unterschiedlichen Organen stattfindet.

Da die Pränataldiagnostik wegen eines in Frage kommenden Abbruchs der Schwangerschaft so früh wie möglich erfolgen sollte, erscheint die Isolierung nukleierter Erythrocyten zur Entwicklung einer pränataldiagnostischen Methode am vielversprechensten.

Es ist auch bekannt, daß das Blut normaler Erwachsener keine kernhaltigen, sondern nur kernlose Erythrocyten enthält, so daß kernhaltige Erythrocyten spezifisch für das fetale Blut sind (Holzgreve W, Garritsen HSK, Gänshirt-Ahlert D: Fetal cells in maternal circulation. J. Reprod Med 37: 410, 1992).

In jüngerer Zeit wurde eine Methode publiziert, die durch Verwendung eines Zweistufengradienten aus fetalem Vollblut mononukleäre weiße Blutkörperchen von nukleierten roten Blutkörperchen abtrennen kann (Bhat MM, Bieber M, Teng NNH: 0ne step separation of human fetal lymphocytes from nucleated red blood cells. J Immun Meth 131 : 147. 1990). In Abwandlung dieser Technik benutzt die Erfindung einen effektiveren Dreifachgradienten, um nukleierte Erythrocyten aus mütterlichem Blut voranzureichern. Es entstehen dabei nach Zentrifugation von Vollblut über einen Dreifachgradienten aus Ficoll drei distinkte Zellschichten, von denen die mittlere Zellschicht nukleierte Erythrocyten enthält. Wir konnten nachweisen, daß die Anreicherung dieser Zellen um so effektiver ist, je verdünnter diese im Vollblut vor der Zentrifugation sind.

Untersuchungen an Knochenmarkszellen Erwachsener, die kernhaltige Vorläuferzellen der Erythrocyten enthalten, ergaben, daß diese auf den Zellmembranen einen sogenannten Transferrinrezeptor exprimieren (Horton MA: Expression of transferrin receptors during erythroid maturation. Exp Cell Res 144: 361, 1983). Der Transferrinrezeptor ist ein Membranprotein, das für den Eisentransport in die Zelle verantwortlich ist; er kann mit Hilfe eines spezifischen monoklonalen Antikörpers (anti-CD 71) nachgewiesen werden. Eine konventionelle Methode zur Anreicherung antikörper-markierter Zellen aus einem Zellgemisch ist die Verwendung eines Fluoreszenz-aktivierten Zellsorters (FACS). Zusätzlich zur Antikörpermarkierung werden hierbei die Zellen mit einem Fluoreszenzfarbstoff markiert. Das FACS-Gerät kann die fluoreszierenden Zellen von den nicht markierten Zellen unterscheiden und voneinander trennen. Mit Hilfe dieser Methode ist es in jüngster Zeit gelungen, fetale Zellen aus mütterlichem Blut anzureichern (Bianchi DW, Flint AR, Pizzimenti MF etal: Isolation of fetal DNA from nucleated erythrocytes in maternal blood. PNAS 87: 3279. 1990; Price J0, Elias S, Wachtel SS: Prenatal diagnosis with fetal cells isolated from maternal blood by multiparameter flow cytometry. Am J Obstet Gynecol 165: 1731. 1991). Allerdings ist ein FACS Gerät sehr kostspielig (ca. DM 800.000,--) und kann nur von einem dafür spezialisierten langjährig ausgebildeten Mitarbeiter bedient werden.

Da durch diesen erheblichen finanziellen und personellen Aufwand der Einsatz eines FACS Gerätes bei pränataldiagnostischen Routineuntersuchungen einen erheblichen Nachteil mit sich bringen würde, wird gemäß der Erfindung vorgeschlagen, die antikörper-markierten Zellen mit Hilfe eines in jüngerer Zeit entwickelten magnetisch-aktivierten Zellsorters (Miltenyi A., Müller W., Weichel A.: High Gradient magnetic cell separation with MACS - Cytometry 11:231. 1990.) anzureichern. Bei dieser Technik werden die antikörper-markierten Zellen zusätzlich mit magnetischen Beads beladen. Der MACS besteht aus einem starken Magneten, in dessen Feld eine mit Stahlwolle gefüllte Spritze eingebracht wird. Über diese wird das Zellgemisch nach Antikörper- und Beads-Markierung gegeben und die negativen, nicht mit magnetischen Beads beladenen Zellen werden herausgewaschen, während die positive Zellfraktion an der Stahlwolle haften bleibt. Anschließend wird die Spritze aus dem Magnetfeld entfernt und die Positivfraktion, die die markierten Zellen enthält, herausgespült. Die Verwendung des magnetisch aktivierten Zellsorters bietet den Vorteil, daß er sehr einfach zu bedienen ist und die Kosten der Anschaffung (ca. DM 10.000,--) weit unter denen eines FACS Gerätes liegen.

Als Nachweis der kindlichen Herkunft der angereicherten Zellen diente die Fluoreszenz in situ Hybridisierung (FISH). Mit dieser Technik können fluoreszenzmarkierte Gensonden, die spezifisch für ein Chromosom sind, auf einem Objektträger mit der DNA fixierter Kerne hybridisiert werden. Die Spezifität der DNA-DNA Hybridisierung erlaubt einen spezifischen Nachweis der Gene in den DNA-haltigen Kernen. Unter dem Fluoreszenzmikroskop ist eine erfolgte Hybridisierung durch ein distinktes fluoreszierendes Signal in den Kernen erkennbar. Bei einem normalen menschlichen Chromosomensatz sind mit einer chromosomen-spezifischen Probe der Autosomen (Nicht-Geschlechtschromosomen) zwei distinkte Signale in den Kernen zu erkennen. Bei einem aberranten Chromosomensatz mit einem überzähligen Chromosom (Aneuploidie) kann man mit dieser Methode durch eine chromosomen-spezifische Gensonde 3 Signale nachweisen. Wir verwendeten die Fluoreszenz in situ Hybridisierung zum Nachweis einer kindlichen Aneuploidie nach Anreicherung fetaler Zellen aus dem Blut schwangerer Frauen, die einen Feten mit bekannter Chromosomenanomalie austrugen.

Diese Methode wurde gewählt, weil Aneuploidien die häufigste Ursache für genetische Fehlbildungen darstellen und einer Diagnose dieser Anomalien in der Schwangerschaft daher eine große Bedeutung zukommt. Besonders wichtig ist der Nachweis einer Trisomie der Chromosomen 13, 18 und 21, sowie Aneuploidien der Chromosomen X und Y. Dieses sind die einzigen Trisomien, die zur Geburt eines Feten führen können, die allerdings in allen drei Fällen schwer geschädigt sind. Eine wenig risikoreiche Technik zur Aspiration fetalen Gewebes aus mütterlichem Blut würde daher eine Screeninguntersuchung auf eine dieser häufigsten genetischen Erkrankungen auch bei jungen Frauen ermöglichen.

Die Erfindung wird nachstehend in Verbindung mit den Tabellen und den Abbildungen an einem Ausführungsbeispiel erläutert. Es zeigt:
- Tab. 1:: Schwangerschaftsgeschichte, Probenentnahme und Ergebnisse,
- Tab. 2:: Auswertun der Anreicherung von nukleierten Erythrocyten,
- Tab. 3:: Hybridisierungssignale in Zellen aus mütterlichem Blut,
- Abb. 1:: Prozentsatz der Zellen mit drei Signalen und
- Abb. 2:: Differentielle Zellzählungen.

### Blutproben

Vierzig ml heparinisiertes Vollblut wurde von neun schwangeren Frauen abgenommen. Drei dieser Frauen trugen einen Feten mit einer Trisomie 18 aus (Tab. 1).

Zur Kontrolle wurden zusätzlich jeweils 10 ml heparinisiertes Nabelschnurblut von vier normalen Neugeborenen und einem Neugeborenen mit einer Trisomie 18 untersucht. Nabelschnurblut ist immer kindlicher Herkunft.

Als Kontrolle wurden jeweils 40 ml Blut von drei nichtschwangeren Frauen und drei Männern untersucht.

Das Blut sollte vor der Verarbeitung nicht älter als 12 Stunden sein.

Zur gesonderten Bestimmung der Effektivität des Dreifachgradienten wurde Nabelschnurblut in Erwachsenenblut in einer Serie so verdünnt, daß das Verhältnis kernhaltiger Zellen von 1 : 10 bis 1 : 50.000 abnimmt, und anschließend ein Dreifachgradient mit jeder einzelnen Verdünnung durchgeführt.

### Dreifachgradient

Jeweils 2 ml heparinisiertes Vollblut werden mit 4 ml PBS Puffer (8.42 g NaCl, 0.2 g KCl, 0.299 g KH₂PO₄, 0.92 g NaHPO₄, ad 1000 ml aqua dest.) gemischt und in ein 12 ml Zentrifugenröhrchen gegeben. Mit einer langen Kanüle werden nacheinander vorsichtig jeweils 2 ml Ficoll-Histopaque (Sigma, München) folgender Dichten unterschichtet: Ficoll-Histopaque 1077, 1110 und 1119. Das Ficoll der Dichte 1110 wird aus entsprechenden Anteilen der Dichten 1077 und 1119, die beide bei Sigma (München) erhältlich sind, gemischt. Die Gradienten werden 30 min bei 550g zentrifugiert. Es sind dann folgende drei distinkte Zellschichten in dem klaren Ficollüberstand zu sehen:

Eine obere Schicht, die Lymphocyten und Monozyten enthält,

eine mittlere Bande, die nukleierte Erythrocyten enthält,

eine untere Bande mit neutrophilen Granulozyten.

Bei Blut aus Schwangeren ist die Zellzahl der angereicherten Zellen so gering, daß sie nicht sichtbar ist, ihre Lage ist jedoch an dem übergang der beiden Ficollschichten unterschiedlicher Dichte gut zu erkennen. Am Boden des Zentrifugationsröhrchens befindet sich ein dicker roter Saum kernloser Erythrocyten. über den Ficollschichten liegt eine gelbliche Schicht mit Blutserum. Nacheinander werden mit einer 10 ml Spritze und einer TSK Subra Einmalkanüle, 0.9 x 120 (Ehrhardt, Geislingen) das Blutserum, die obere, die mittlere und untere Bande von dem Gradienten abgenommen. Die unterste Zellschicht mit den nicht kernhaltigen Erythrocyten wird verworfen. Die mittlere Bande, die die angereicherten nukleierten Erythrocyten enthält, wird mit 5 ml PBS-Puffer versetzt und drei mal mit PBS Puffer gewaschen, d. h. jeweils 8 min. bei 550 g zentrifugiert und das Zellpellet erneut mit PBS Puffer versetzt. Danach wird die Zellsuspension auf ca. 2 ml eingeengt, 100 µl entnommen und in einer Zytozentrifuge (Shandon, Frankfurt) 5 min. bei 500 g auf einen Objektträger zentrifugiert. Auf dem Objektträger werden die Blutzellen differentiell angefärbt mit einer Diff-Quick Färbelösung (Merz+Dade, Düdingen, CH). Diese Färbung erlaubt die mikroskopische Unterscheidung der verschiedenen Blutzellen voneinander. Es wurden auf diese Weise jeweils 100 Zellen nach Separierung durch den Gradienten ausgezählt, um den Erfolg der Anreicherung nukleierter Erythrocyten in der mittleren Zellschicht zu überprüfen.

### Antikörperfärbung

In einer Thomakammer wurde die Zellzahl der mittleren Bande nach dem Dreifachgradienten bestimmt.

Bei der Verwendung von Nabelschnurblut wurde ein Aliquot dieser angereicherten Zellsuspension zur Antikörpermarkierung eingesetzt, das einer Zellzahl von 10⁷ Zellen entsprach.

Die Zellen wurden 10 min. bei 200 g zentrifugiert und das Pellet in 50 µl PBS Puffer, der 10 % Serum enthält, aufgenommen. Das Serum wird nach dem Dreifachgradienten entnommen und vor der Verwendung 45 min. bei 56° C inkubiert. Die Verwendung des Serums bei der Antikörperinkubation verhindert die unspezifische Markierung von Monozyten.

Zu den 50 µl der Zellsuspension werden 50 µl "anti-CD71" (Becton-Dickenson) gegeben und das Gemisch 10 min. bei 8° C inkubiert. Anschließend werden 20 µl rat-anti-mouse IgG 2 (a+b) magnetische Microbeads (Miltenyi Biotech, Bergisch-Gladbach) dazugegeben und das Gemisch weitere 15 min. bei 8° C inkubiert. Nach Zentrifugation (200 g, 10 min.) wird der Überstand abgenommen und die Zellen in 500 µl PBS/0.5 % BSA (bovine serum albumine, Fluka, Buchs) resuspendiert.

Eine A2 Säule der Firma Milenyi Biotech, die mit Stahlwolle gefüllt ist und sich zur Separation von ca. 10⁷ Zellen eignet, wird für die Zelltrennung wie folgt vorbereitet: An das untere Ende der Säule wird ein Dreiwegehahn angeschlossen und an den seitlichen Eingang eine mit 70 %igem Ethanol gefüllte 10 ml Spritze angesetzt. Das Ethanol wird von unten in die Säule hineingedrückt, bis der Flüssigkeitsspiegel den oberen Rand der A2 Säule erreicht hat. Der Dreiwegehahn wird geschlossen und von oben eine 100 ml Spritze mit Gewinde auf die Säule aufgeschraubt, die Aqua dest. enthält. Der Dreiwegehahn wird geöffnet, so daß die Säule nach unten auslaufen kann und mit Aqua dest. durchgespült wird. Anschließend wird die Säule mit PBS/0.5 % BSA durchgespült und das P3S/BSA Gemisch durch rechtzeitiges Schließen des Hahns in der Säule belassen, so daß der Puffer ca. 1 cm über der Stahlwolle steht. Durch diese Behandlung gelingt es, die Säule luftblasenfrei mit PBS/BSA Puffer zu füllen. Vorhandene Luftblasen würden eine spätere Bindung der Zellen an die Stahlwolle verhindern. Das PBS/BSA Gemisch muß 30 min. in der Säule verbleiben, damit sie mit BSA abgesättigt wird, um unspezifische Bindungen zu verhindern.

Vor der Zellseparation wird die Säule an dem Magneten befestigt und durch Öffnen des Dreiwegehahns der Puffer so weit entfernt, daß der Flüssigkeitsspiegel kurz über der Stahlwolle steht. Von oben werden die 500 µl Zellsuspension nach Antikörperfärbung auf die Säule pipettiert und der Hahn so lange geöffnet, bis der obere Rand des Flüssigkeitsspiegels wieder fast die Stahlwolle erreicht hat. Danach werden 6 Fraktionen von jeweils 500 µl PBS/0.5 % BSA hinzugegeben und die Säule damit gewaschen. Diese Negativfraktion wird mit einer Nadel der Stärke 24 G eluiert und in einem Zentrifugenröhrchen gesammelt. Danach wird der Dreiwegehahn geschlossen und die Säule aus dem Magneten entfernt. Unten an dem Hahn wird seitlich eine Spritze mit PBS/0.5 % BSA befestigt. Nach Öffnen des Hahns wird der Puffer mit der Spritze so weit in die Säule gepreßt, daß die Zellsuspension den oberen Säulenrand erreicht. Nach Schließen des Dreiwegehahns wird diese wieder in dem Magneten befestigt und eine Nadel der Stärke 22 G unten angebracht. Die Säule wird dann erneut 6 mal mit jeweils 500 µl PBS/0.5 % BSA eluiert, diese Fraktion wird als Waschfraktion gesammelt. Nach dem Schließen des Dreiwegehahns wird die Säule aus dem Magneten entfernt, bis zum oberen Rand mit PBS/0.5 % BSA Puffer gefüllt und oben eine ebenfalls mit 3 ml PBS/BSA Puffer gefüllte Spritze aufgesetzt. Nach Entfernen der Nadel wird durch Druck auf die Spritze die "Positivfraktion" nach unten ausgespült.

Von allen drei Fraktionen "Negativ-", "Wasch-" und Positivfraktion" wurden Aliquots zur Zellzahlbestimmung und Zytozentrifugation entnommen. Im Anschluß an die Zytozentrifugation wurden die Objektträger differentiell angefärbt und bei Nabelschnurblut jeweils 100 Blutzellen ausgezählt, um die erfolgte Anreicherung zu überprüfen. Bei Schwangerenblut wurden alle Zellen des gesamten Zytospins ausgezählt.

### Fluoreszenz In Situ Hybridisierung

Die positive Fraktion der magnetischen Zellseparation wurde zur Isolation der Interphasekerne 10 min. bei 200 g zentrifugiert, das Pellet in 5 ml 75 mM KCL resuspendiert und 10 min. bei 37° C inkubiert. Nach Zentrifugation (10 min. 150 g) wurde das Zellpellet vorsichtig in 5 ml Methanol/Eisessig (3 : 1/v : v) resuspendiert und 10 min. bei 200 g zentrifugiert. Das Pellet wurde in dem Rücklauf des Methanol/Eisessig resuspendiert und mit einer Glaspasteurpipette auf einen Objektträger aufgetropft.

Die anschließende Fluoreszenz in situ Hybridisierung mit einer DNA Sonde, die spezifisch für das Chromosom 18 ist, erfolgte mit einem Kit der Firma 0ncor (Gaithersburg, USA) nach den Anweisungen des Herstellers.

Die Kerne wurden nach der in situ Hybridisierung unter einem Zeiss Fluoreszenz-Mikroskop ausgewertet und mit einem Kodak Ectachrom 64 ASA Film fotografiert.

### Ergebnisse

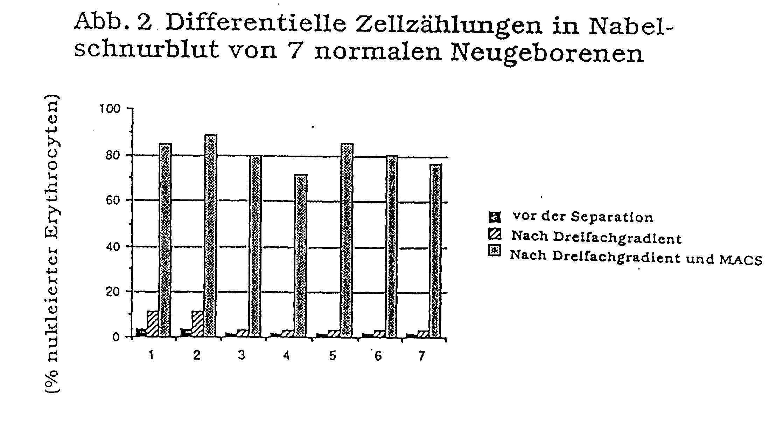

Die Ergebnisse differentieller Zellzählungen vor und nach dem Dreifachgradienten und nach MACS von sieben Nabelschnurblutproben sind in der Abb. 2 dargestellt. Die starke Anreicherung nach der kombinierten Anwendung beider Methoden ist klar erkennbar. Die Separationseffizienz des Dreifachgradienten steigt signifikant an, wenn die Zahl der nukleierten Erythocyten in der Vollblutprobe abnimmt. Dies konnte in Untersuchungen von Dreifachgradienten mit Verdünnungsreihen von Nabelschnurblut in Erwachsenenblut nachgewiesen werden (Tab. 2). Man kann also davon ausgehen, daß bei der Separation von Schwangerenblut die Effektivität des Dreifachgradienten deutlich höher ist als dies bei der Anreicherung aus Nabelschnurblut der Fall ist.

Tabelle 1 zeigt die Anzahl nukleierter Erythrocyten, die durch die Kombination beider Separationsmethoden aus dem Blut von neun Schwangeren isoliert werden konnten.

Nach Separation von drei männlichen Kontrollblutproben und drei Blutproben von nichtschwangeren Frauen konnten keine nukleierten Erythrocyten nachgewiesen werden.

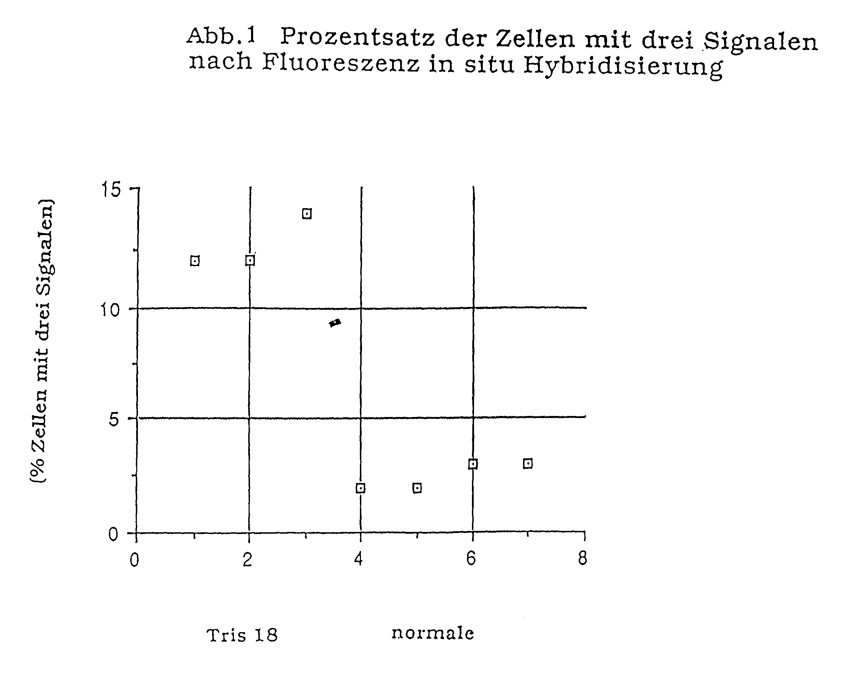

Bei drei der untersuchten Schwangerschaften wurde bei bekannter Trisomie 18 des Feten (Tab. 1) im Anschluß an die Zellseparation eine Fluoreszenz in situ Hybridisierung mit einer Chromosom 18 spezifischen DNA Sonde durchgeführt. In allen drei Fällen war der Prozentsatz der Zellen mit drei Signalen im Vergleich zu Kontrollen signifikant erhöht (Tab. 3). Der Anteil der Zellen mit drei Signalen lag bei 12-, 12- und 14 % in den Fällen mit Trisomie 18. Bei vier Blutproben normaler Neugeborener lag der mittlere Prozentsatz der trisomen Zellen nach FISH bei 2.5 %. Abbildung 1 stellt diese Daten aus drei Schwangerschaften und vier Nabelschnurblutkontrollen dar.

Die beiden Verteilungskurven zeigen keine überlappung und der Unterschied ist hoch signifikant. Bei Patientin 3 untersuchten wir Nabelschnurblut, das von dem Feten mit Trisomie 18 in utero gewonnen wurde und fanden 70 % der Zellen mit drei Signalen.

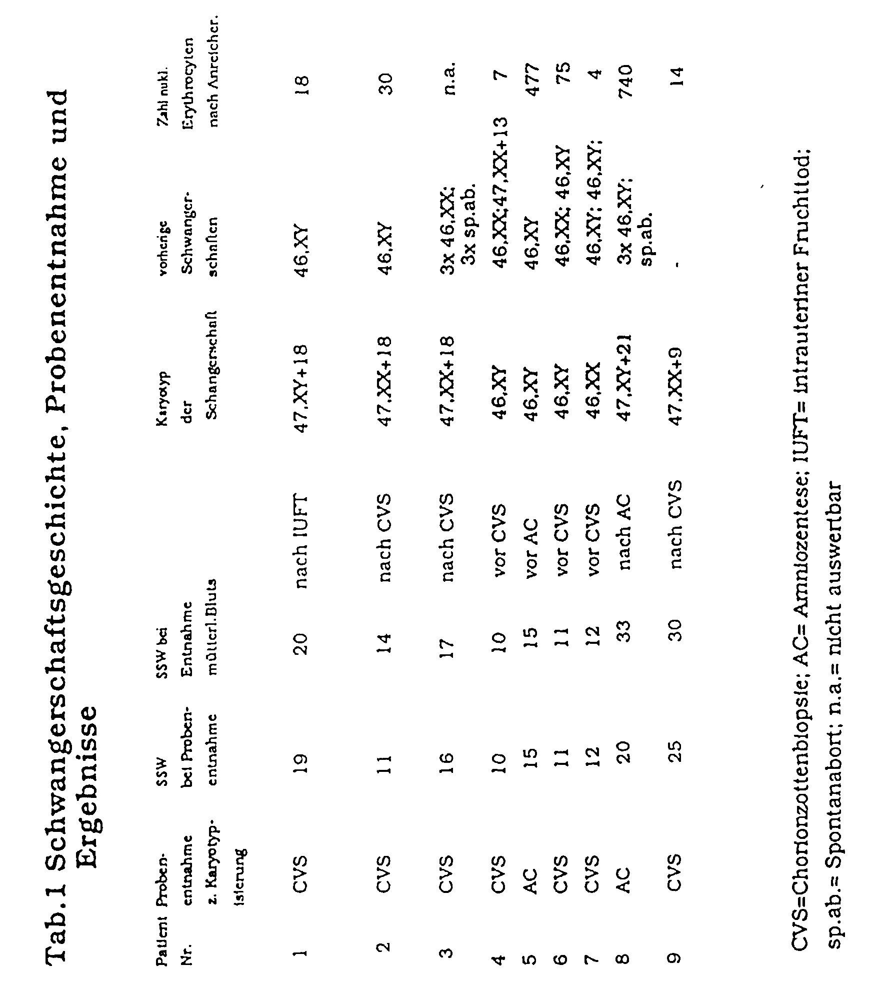

**Tab. 2**

| Auswertung der Anreicherung von nukleierten Erythrocyten nach Dreifachgradient aus Nabelschnurblut verdünnt in Erwachsenenblut. Die Zahl der nukleierten Erythrocyten wurde auf differentiell gefärbten Zytospins bzw. Blutausstrichen ausgewertet. | | | | | | | | |
|---|---|---|---|---|---|---|---|---|
| % nukleierte Erythrocyten im Blutausstrich | % nukleierte Erythrocyten nach Dreifachgradient Nabelschnurblut verdünnt in weiblichem Blut | | | | | | | |
| Nabelschnurblut unverdünnt | 1:10 | 1:50 | 1:100 | 1:500 | 1:1000 | 1:50000 | 1:10000 | 1:500000 |
| 4 | 8 | 2 | 1 | 1 | 0.2 | 0 | 0.1 | 1 in 2 OT |
| | | | | | | | | |
| 17 | 5 | 1 | 0 | 3 | 0 | 0 | 0 | 3 |
| OT: Objektträger | | | | | | | | |

**Tab. 3**

| Hybridisierungssignale in Zellen aus mütterlichem Blut von 3 Schwangerschaften mit Trisomie 18, einer Nabelschnurblutprobe eines Feten mit Trisomie 18 und 4 Nabelschnurblutproben von Feten mit normalem Chromosomensatz. | | | | | | | |
|---|---|---|---|---|---|---|---|
| Patientin | % und Anzahl der Hybridisierungssignale | | | | | | Gesamtzellzahl |
| | 0 | 1 | 2 | 3 | 4 | 5 | |
| No. 1 (Tris 18) | 9%(10) | 12%(13) | 65%(72) | 12%(13) | 2%(2) | 0% | 110 |
| No.2 (Tris 18) | 6%(6) | 7%(7) | 68%(64) | 12%(11) | 2%(2) | 0% | 90 |
| No. 3 (Tris 18) | 6%(6) | 3%(3) | 70%(70) | 14%(14) | 1%(1) | 0% | 94 |
| No.3 Nabelschnurblut (Tris 18) | 0% | 0% | 22%(18) | 70%(56) | 7%(5) | 1%(1) | 80 |
| Kontrolle 1 Nabelschnurblut (46,XY) | 3%(7) | 8%(16) | 78%(161) | 2%(4) | 2%(2) | 0% | 190 |
| Kontrolle 2 Nabelschnu-r blut (46,XY) | 9% (5) | 9% (5) | 78%(42) | 2%(1) | 2%(1) | 0% | 54 |
| Kontrolle 3 Nabelschnurblut (46,XY) | 3%(6) | 6%(12) | 89%(177) | 3%(3) | 2%(2) | 0% | 200 |
| Nabelschnurblut (46,XX) | 5%(9) | 5%(9) | 88%(176) | 3%(5) | 1%(1) | 0% | 200 |

## Patentansprüche

1. Verfahren zur Pränataldiagnostik genetischer Anomalien, das die Kombination folgender Schritte umfaßt:
a) Voranreicherung kindlicher Zellen in Form von nukleierten Erythrocyten aus mütterlichem Vollblut durch einen Dreifach-Ficoll-Gradienten,
b) Markierung der kindlichen Zellen mit einem monoklonalen Antikörper und/oder Markierung mütterlicher Zellen mit entsprechenden spezifischen Antikörpern mit magnetischen Beads und Anreicherung der markierten fetalen Zellen bzw. Depletion der mütterlichen Zellen durch magnetische Separation,
c) Nachweis kindlicher genetisch bedingter Anomalien durch molekulargenetische Nachweismethoden.

2. Verfahren nach Anspruch 1, dadurch gekennzeichnet, daß die Markierung der kindlichen Zellen mit einem monoklonalen Antikörper gegen den Transferrinrezeptor erfolgt.

3. Verfahren nach Anspruch 1 oder 2, dadurch gekennzeichnet, daß als monoklonaler Antikörper ein spezifischer "anti - CD71" (Becton-Dickenson) verwendet wird.

4. Verfahren nach einem der vorhergehenden Ansprüche, gekennzeichnet durch den Nachweis kindlicher Chromosomenstörungen durch Fluoreszenz in situ Hybridisierung mit chromosomen-spezifischen DNA Sonden.

5. Verfahren nach einem der vorhergehenden Ansprüche, dadurch gekennzeichnet, daß die Anreicherung der markierten fetalen Zellen durch einen magnetisch aktivierten Zell Sorter (MACS) erfolgt.

## Claims

1. A method for prenatal diagnosis of genetic abnormalities, which comprises a combination of the following steps:
a) pre-enriching of infantile cells in the form of nucleated erythrocytes from maternal whole blood by a triple Ficoll gradient,
b) labelling of the infantile cells with a monoclonal antibody and/or labelling of maternal cells with corresponding specific antibodies with magnetic beads and enriching of the labelled foetal cells or depletion of the maternal cells by magnetic separation,
c) detecting of genetically determined infantile abnormalities by molecular-genetic detection methods.

2. A method according to claim 1, characterised in that labelling of the infantile cells is effected with a monoclonal antibody against the transferrin receptor.

3. A method according to claim 1 or claim 2, characterised in that a specific "anti-CD71" (Becton-Dickenson) is used as the monoclonal antibody.

4. A method according to any one of the preceding claims, characterised by the detection of infantile chromosome abnormalities by fluorescence in situ hybridisation with chromosome-specific DNA probes.

5. A method according to any one of the preceding claims, characterised in that enriching of the labelled foetal cells is effected by a magnetically activated cell sorter (MACS).

## Revendications

1. Procédé pour le diagnostic prénatal d'anomalies génétiques, qui comporte la combinaison des étapes suivantes :
a) enrichissement préalable de cellules foetales sous forme d'érythrocytes nucléés provenant d'un sang total maternel, par un gradient triple de Ficoll,
b) marquage des cellules foetales avec un anticorps monoclonal et/ou marquage des cellules maternelles avec des anticorps spécifiques correspondants à l'aide de perles magnétiques, et enrichissement, par séparation magnétique, des cellules foetales marquées ou épuisement des cellules maternelles,
c) détection des anomalies foetales d'origine génétique, par des méthodes de détection du génie génétique.

2. Procédé selon la revendication 1, caractérisé en ce que le marquage des cellules foetales s'effectue avec un anticorps monoclonal contre le récepteur de la transferrine.

3. Procédé selon la revendication 1 ou 2, caractérisé en ce qu'on utilise en tant qu'anticorps monoclonal un "anti-CD71" spécifique (Becton-Dickenson).

4. Procédé selon l'une des revendications précédentes, caractérisé par la détection de troubles chromosomiques de l'enfant par hybridation in situ par fluorescence avec des sondes à ADN spécifiques de chromosome.

5. Procédé selon l'une des revendications précédentes, caractérisé en ce que l'enrichissement des cellules foetales marquées s'effectue par un trieur de cellules à activation magnétique (MACS).
